Europäisches Patentamt

European Patent Office

Office européen des brevets

(19)

(11)  EP 0 762 881 B1

(12)  EUROPEAN PATENT SPECIFICATION

(45) Date of publication and mention
of the grant of the patent:
**17.03.2004  Bulletin 2004/12**

(21) Application number: **95917611.6**

(22) Date of filing: **24.04.1995**

(51) Int Cl.[7]: **A61K 39/40**, A61K 35/74,
A61K 31/711, C07K 16/04,
C07K 16/12, C07K 14/245,
C07K 14/705

(86) International application number:
**PCT/US1995/004896**

(87) International publication number:
**WO 1995/031984 (30.11.1995 Gazette 1995/51)**

(54) **USE OF ACTIVE AGENTS FOR THE MANUFACTURE OF MEDICAMENTS FOR THE TREATMENT OF RHEUMATOID ARTHRITIS**

VERWENDUNG VON WIRKSTOFFEN ZUR HERSTELLUNG VON ARZEINMITTELN ZUR BEHANDLUNG DER RHEUMATISCHEN ARTHRITIS

UTILISATION D'AGENTS ACTIFS POUR LA PREPARATION DE MEDICAMENTS POUR LE TRAITEMENT DE L'ARTHRITE RHUMATOIDE

(84) Designated Contracting States:
**AT BE CH DE DK ES FR GB GR IE IT LI LU MC NL PT SE**

(30) Priority: **20.05.1994  US 246988**

(43) Date of publication of application:
**19.03.1997  Bulletin 1997/12**

(73) Proprietor: **The Regents of the University of California
Oakland, CA 94607-5200 (US)**

(72) Inventors:
• **CARSON, Dennis, A.
Del Mar, CA 92014 (US)**
• **SALVATORE, Albani
San Diego, CA 92109 (US)**

(74) Representative: **Goldin, Douglas Michael
J.A. KEMP & CO.
14 South Square
Gray's Inn
London WC1R 5JJ (GB)**

(56) References cited:
**WO-A-90/14835          US-A- 4 732 757**

• **GEORGESCU C: "STUDY ON THE THERAPEUTICAL EFFECT OF A WIDE SPECTRUM ANTIBIOTIC KANAMYCIN IN RHEUMATOID ARTHRITIS." REV ROUM MED INTERNE, (1969) 6 (6), 411-423. CODEN: RRMIAI. ISSN: 0035-3973., XP002095474**
• **TAKEUCHI, F. ET AL: "Susceptibility epitope on the HLA-DR.beta. chain for rheumatoid arthritis and the effect of the positivity on the clinical features" J. IMMUNOGENET. (1989), 16(6), 475-83 CODEN: JIMGAV;ISSN: 0305-1811, XP002095475**
• **THE JOURNAL OF PEDIATRICS, Vol. 124, No. 4, April 1994, ALBANI et al., "Immune Responses to the Escherichia Coli dnaJ Heat Shock Protein in Juvenile Rheumatoid Arthritis and Their Correlation With Disease Activity", pages 561-565.**
• **J. CLIN. INVEST., Vol. 89, January 1992, ALBANI et al., "The Susceptibility Sequence to Rheumatoid Arthritis is a Cross-Reactive B Cell Epitope Shared by the Escherichia Coli Heat Shock Protein dnaJ and the Histocompatibility Leukocyte Antigen DRB10401 Molecule", pages 327-331.**

**Description**

**BACKGROUND OF THE INVENTION**

1. *Field of the Invention*

The invention relates to the control and prevention of autoimmune disease, in particular rheumatoid arthritis. More specifically, the invention relates to the use reagents which reduce or prevent the response of a host to proteins which include an amino acid sequence (Q(K/R)RAA) that is homologous to a sequence contained in certain HLA proteins.

2. *History of the Prior Art*

In humans, autoimmune diseases such as rheumatoid arthritis tend to be associated with particular HLA specificities. Rheumatoid arthritis (RA) in particular is presently believed to be associated on a genetic level with the Class II HLA haplotypes DW4, DW14, DW15 (all with DR4 specificity) and/or DR1. Each of these haplotypes include an amino acid sequence which is commonly referred to as the "susceptibility sequence" (hereafter, "RA susceptibility sequence";see, SEQ.ID.NOs: 1 and 2). The RA susceptibility sequence is known to vary at one amino acid; to wit, QRRAA and QKRAA (hereafter, "Q(R/K)RAA"). More than 90% of patients with seropositive RA have also been found to have HLA DR antigens with the RA susceptibility sequence in the third hypervariable region of the molecule.
The QRRAA variant of the susceptibility sequence has been identified on HLA haplotypes DW14, DW15 and DR1. The QKRRA variant has been identified on HLA haplotype DW4. Homologs of the variants have also been identified in the Epstein-Barr virus glycoprotein gpl10, as well as the dnaJ heat shock proteins from *Escherichia coli,* as well as the bacterial species *Klebsiella, Proteus Salmonella,* and *Lactococcus.*
Based on the substantial degree of conservation among prokaryotic dnaJ proteins, it can be expected that the RA susceptibility sequence is present in dnaJ produced by other microbial species (see, e.g., *J. Bact.*, 175: 1637-1644 (1993)). Further, based on reports of sequence homology among eukaryotic dnaJ proteins with other eukaryotic and prokaryotic dnaJ proteins, it would not necessarily be surprising to find the RA susceptibility sequence in eukaryotic dnaJ, although it has not as yet been identified in such proteins (see, e.g., Silver, *et al. Cell*, 74:5-6 (1993)).
In 1992, a report was published which supported the theory that the dnaJ protein played a role in the onset of rheumatoid arthritis (Albani, *et al*., *J. Clin. Invest.*, 89:327-331 (1992)). The authors expressed and purified recombinant dnaJ (rdnaJ). The rdnaJ was specifically bound by antisera raised in rabbits against the RA susceptibility sequence from DW4 proteins. Antisera raised against rdnaJ also bound the intact DW4 protein as well as DW4 homozygous B lymphoblasts.
Two hypotheses for the role of the RA susceptibility sequence on HLA DR1 and DW4 prevailed at the time of the above-described report. The first postulated that.the RA susceptibility sequence controlled binding of an unknown arthritogenic peptide. The second postulated that the sequence bound antigen receptors on T cells, thereby influencing the expansion or deletion of a particular T cell population. Specifically, under the latter hypothesis, the RA susceptibility sequence is believed to be part of a peptide amino acid sequence presented to T cells. This amino acid sequence is apparently recognized by the T cells as non-self rather than self. Under both theories, the onset of RA is believed to emanate at least in part from a failure of the local immune system of the gut to produce secretory immunoglobulins ("IgA") against harmful microorganisms.
With the recognition that microorganisms produce peptides having the RA susceptibility sequence, a third theory emerged which postulated that the autoimmune response involved in RA was "learned" as a result of the host's immune response to microbial RA susceptibility sequence peptides. Based on this theory, a therapeutic modality was proposed which would vaccinate humans against RA by administering synthetic conjugates of immunogenic forms of RA susceptibility sequence peptides thereto (see, published PCT application, WO 9014835 (filed 5/31/90), Carson, *et al.*, inventors). The efficacy of the proposed vaccines has not yet been tested in humans.
Another therapeutic modality for controlling the immune response to bacterial antigens believed to be implicated in the onset of RA has been proposed in U.S. Patent No. 4,732,757 to Stolle, *et al*. Stolle, *et al*. proposed administering IgG antibodies raised in cow's milk against a broad spectrum of intact bacteria which typically reside in the human gastrointestinal tract. The IgG tested by Stolle, *et al*., were raised against a mixture of intact organisms without targeting specific bacterial antigens. This patent issued prior to the identification of the RA susceptibility sequence in certain bacterial heat shock proteins, including dnaJ. The antisera described by Stolle, *et al.* were not, therefore, directed toward RA susceptibility sequence peptides.
None of these approaches to treating RA specifically target arthritogenic peptides before the peptides are presented to the systemic immune system of an RA patient. A need, therefore, exists for a treatment which will not only ameliorate systemic responses by an RA patient to arthritogenic peptides, but will also prevent exposure of the patient to the arthritogenic peptides through the release of such peptides from the patient's GI tract into systemic circulation. Ideally, such treatments will be provided either before the patient develops RA or in the early stages of the disease; thus, a need also exists for a method of identifying persons who are genetically predisposed to develop RA.

The present invention addresses each of these needs.

## SUMMARY OF THE INVENTION

The invention comprises several ways for ameliorating or avoiding host sensitization to microbial arthritogenic peptides, particularly dnaJ. For purposes of this disclosure, the phrase "arthritogenic peptides" will, unless context otherwise requires, refer to proteins and peptide fragments thereof which contain the RA susceptibility sequence, preferably those of microbial origin.

In one aspect of the invention, IgG, IgA are raised (preferably in milk) against the arthritogenic peptides (preferably derived from rdnaJ) and are to be administered (preferably by enteral routes and preferably as Fab fragments of the Ig) to an RA patient. In another aspect of the invention, bacteria capable of producing arthritogenic peptides are substantially eliminated from the gastrointestinal tract of an RA patient then replaced with bacteria engineered to be incapable of expressing the RA susceptibility sequence.

The aspects of the invention may be employed separately or in combination. The invention also encompasses uses for anti-arthritogenic peptide antibodies, including anti-dnaJ antibodies.

The invention also comprises the screening of candidates for the above-described therapies by detecting *in vitro* cellular immune responses to an arthritogenic peptide.

The details of the preferred embodiment of the present invention are set forth in the accompanying drawings and the description below. Once the details of the invention are known, numerous additional innovations and changes will become obvious to one skilled in the art.

## BRIEF DESCRIPTION OF THE DRAWINGS

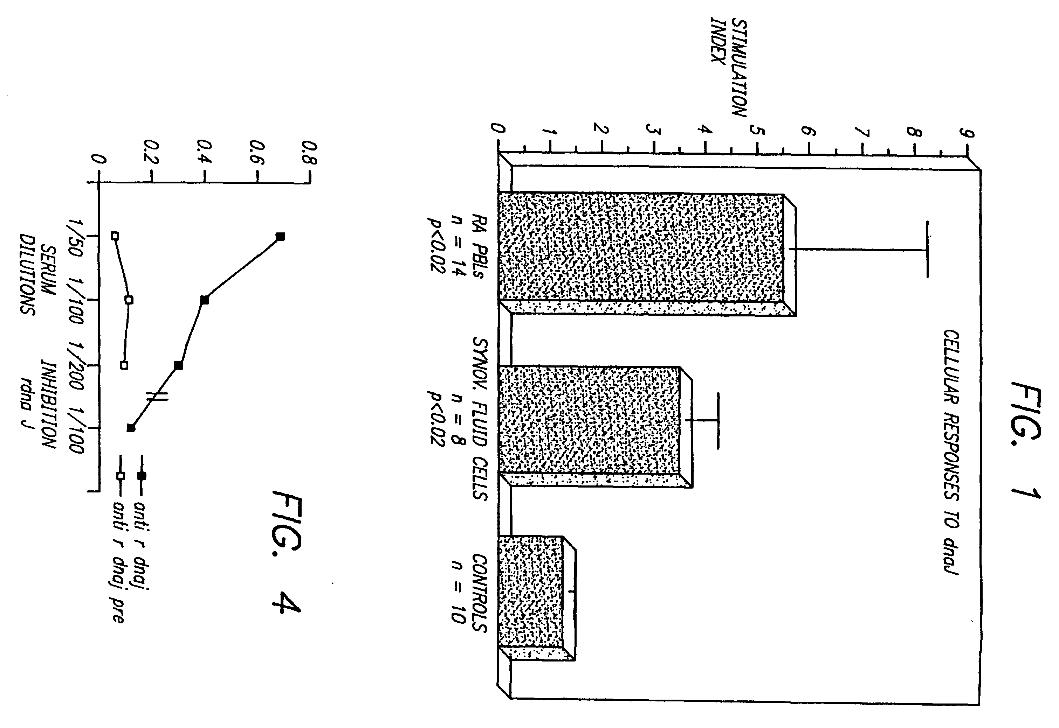

FIGURE 1 quantifies proliferation of cultured lymphocytes from 22 patients with early, untreated RA ("early stage" RA) following exposure of the cells to rdnaJ. The responses shown by bars in the graph of FIGURE 1 are expressed in terms of a lymphocyte stimulation index; i.e., cpm detected in cultures with rdnaJ/cpm detected in cultures without dnaJ. The control culture consisted of lymphocytes from humans without RA.

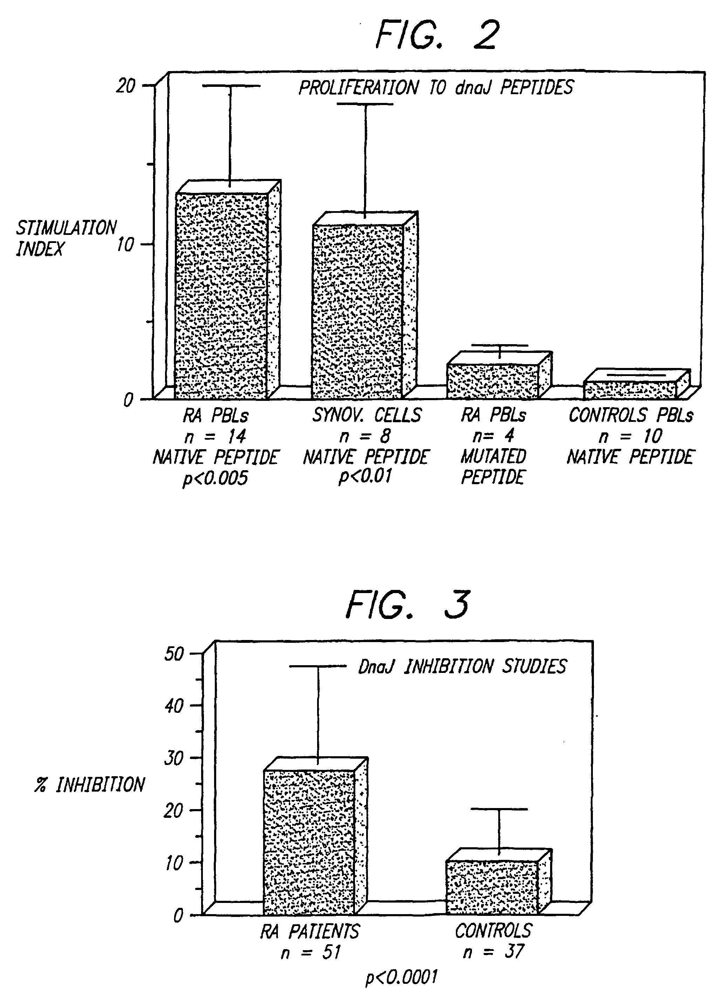

FIGURE 2 quantifies proliferation of cultured lymphocytes from 26 early stage RA patients following exposure of the cells to a synthetic dnaJ peptide having the amino acid sequence Q<u>K</u>RAAYDQYGHAAFE or a mutant dnaJ peptide having the amino acid sequence <u>DER</u>AAYDQYGHAAFE. The control culture consisted of lymphocytes from humans without RA.

FIGURE 3 depicts the results of an inhibition study for inhibition of anti-dnaJ antibodies produced in separate sera samples from 51 early stage RA patients by rdnaJ. The percent inhibition shown by bars in the graph of FIGURE 2 is expressed in terms of the formula:

$$\% \text{ inhibition} = 100 \ \frac{\text{I-OD}_{460} \text{ with rdnaJ)}}{(\text{OD}_{460} \text{ without rdnaJ})}$$

The control sera were obtained from humans without RA.

FIGURE 4 depicts the *in vitro* antibody response of rabbit sera to rdnaJ as measured by an enzyme-linked immunosorbent assay. The results are expressed as the mean OD at 405 nm of duplicate plate wells.

## DETAILED DESCRIPTION OF THE INVENTION

I. THEORY OF THE INVENTION

A discovery underlying this invention is that human patients with early stage RA have abnormally high cellular immune responses to a microbial peptide having the RA susceptibility sequence. This observation leads to the conclusion that RA is triggered or exacerbated by immune responses to sterile fragments of bacterial antigens that are transported to the joints by monocytes. Further, the molecules involved in these immune responses are likely to be cross-reactive with the RA susceptibility sequence on HLA DW4 and DR1, particularly the former.

Although the invention is not limited to or dependent upon the following theory concerning the pathogenesis of RA, the theory is provided as a reasonable explanation of why and how the methods of the invention exploit the immune mechanism likely to be involved in RA. More particularly, the granulation tissue of the rheumatoid pannus is filled with cells of the monocytic lineage. In humans (as opposed to lower animals) these tissue macrophages do not divide efficiently, and must be replenished continually from circulating blood monocytes. It is reasonable to suppose that immune complexes containing dnaJ and IgG antibody to arthritogenic peptide form first in the intestinal mucosal region, and are picked up by circulating blood monocytes with Fc receptors.

Some of these antigen-loaded monocytes then may migrate systemically to the joints. The T lymphocytes from individuals with the RA susceptibility sequence probably have been educated to hyperrespond to dnaJ, such that tiny amounts of antigen would suffice to initiate joint inflammation. Very small quantities of a common bacterial antigen might never be detected by classic immunologic means. Moreover, once established, synovitis could be sustained by the further development of antibodies to the immune complexed IgG (rheumatoid factors), and to degraded components of cartilage and connective tissue. However, at least the early stages of rheumatoid arthritis may depend upon the continual loading of circulating monocytes with IgG immune complexes containing arthritogenic peptides.

The use of the active agent according to the invention is designed to ameliorate or prevent the immune response to arthritogenic peptides described above. In the former approach, early stage RA patients or patients who have a high risk of developing RA are passively immunized with antibodies which will specifically bind arthritogenic peptides, preferably antibodies produced in mammalian milk. In the latter approach, a nonabsorbable antibiotic is to be administered to patients to reduce or eliminate the endogenous bacterial population in the gastrointestinal (GI) tract. This population is then replaced in the GI tract by bacteria which have been engineered to be incapable of expressing the RA susceptibility sequence. Both alternative may be supplemented by an active immunization protocol wherein immunogenic arthritogenic peptides are administered as a vaccine to the patient, alone or in combination with immunostimulatory agents.

## II. REAGENTS AND MEANS FOR USE IN IDENTIFYING SUITABLE CANDIDATES FOR THE THERAPY ACCORDING TO THE INVENTION

### A. Characteristics of Suitable Candidates for the Therapy according to the Invention.

The principal candidates for the therapy according to the invention are individuals who have early stage RA or who are predisposed to develop the disease. In this regard, with one caveat, an individual may be considered to be "predisposed" to develop RA when their HLA molecules contain the RA susceptibility sequence.

The caveat, however, is based on the observation that certain "normal" individuals (i.e., who do not have a family history of RA and do not develop it themselves) may nonetheless have endogenous HLA molecules which bear the RA susceptibility sequence. Yet despite the presence of the RA susceptibility sequence in these individuals, their cellular immune responses to dnaJ may be no different in absolute magnitude from the responses of individuals whose HLA molecules do not bear the RA susceptibility sequence.

It can be presumed from the preceding observation that the development of RA in persons whose HLA molecules bear the RA susceptibility sequence is the result not only of a cellular immune response, but also a humoral immune response. In other words, the development of RA is most likely associated not only with the education of the T cell repertoire to recognize the RA susceptibility sequence as non-self, but also to the production in RA sufferers of anti-RA susceptibility sequence antibodies (see, Example IV and FIGURE 3, showing inhibition of antibody binding in lymphocytes from RA patients by dnaJ, where the magnitude of the inhibition substantially exceeds that observed in lymphocytes from normal individuals). Thus, individuals whose HLA antigens bear the RA susceptibility sequence and who produce anti-RA susceptibility sequence antibodies will, for purposes of this disclosure, be considered to be "predisposed" to RA. A screening method for identification of individuals with early stage RA or who are predisposed to it (the "RA screening method") is described below.

### B. Steps of the RA Screening Method.

The RA screening method will generally comprise the steps of (1) detecting whether the RA susceptibility sequence is present on HLA molecules from an individual (in particular, DW4 molecules), and (2) detecting whether lymphocytes from the individual produce antibodies in response to being exposed to an immunogenic form of an arthritogenic protein or peptide. Alternatively, the screening method may entail only the latter step because if the individual's lymphocytes produce antibodies in response to being exposed to an RA susceptibility sequence protein, the individual may be presumed to be more likely to develop RA. For convenience, the HLA molecules which may bear the RA susceptibility sequence in a given individual will be collectively referred to hereafter as "HLA-DW4" molecules, although the term will be understood to encompass any HLA molecule which may bear the RA susceptibility sequence.

To perform the RA screening method, a biological sample, which may be any fluid or tissue likely to contain lymphocytes, but will preferably be whole peripheral blood or synovial fluid (the "lymphocyte sample"), will be obtained using conventional techniques from the individual to be screened for RA. To determine whether the individual's HLA molecules bear the RA susceptibility sequence, conventional HLA typing techniques well-known to those skilled in the art may be employed. It will be appreciated in this regard that the nucleotide and amino acid sequences for most known HLA antigens have been fairly well characterized and can be readily identified (see, e.g., Marsh, *et al.*, *Tissue Antigens*, 37:181-189 (1991), and Bodmer, *et al.*, *Tissue Antigens,* 37:97-105 (1991)).

For example, with reference to the polynucleotide sequences reported by Marsh and Bodmer, *et al.*, and/or the polynucleotide sequences contained in the Sequence Listing appended hereto, those of ordinary skill in the art will be able to construct oligonucleotide probes which will hybridize to target polynucleotide sequences for detection of specific HLA antigens in a lymphocyte sample. Techniques for construction of suitable probes and performance of hybridization techniques are described in greater detail infra and may be employed by those of ordinary skill in the art without undue experimentation.

Commonly, however, HLA typing is performed serologically, i.e., by using standardized antisera (of defined specificity) to a lymphocyte sample together with complement and observing whether the test cells are killed. HLA typing may also be performed by other conventional immunological techniques such as the "Mixed Lymphocyte Reaction" wherein test lymphocytes are mixed with B lymphocytes of defined HLA specificity. In the Mixed Lymphocyte Reaction, test cells of specificity different than the B cells of known HLA type are stimulated and proliferate. Performance of all of these HLA typing techniques is well within the ordinary level of skill in the art.

The presence of the RA susceptibility sequence on the HLA molecules of an individual may also be inferred from binding of anti-dnaJ or anti-arthritogenic peptide antibodies by cells in the lymphocyte sample. Because class D HLA molecules are also present on macrophages, monocytes and some activated T cells, antibody binding on these cells may also occur at specific epitopes.

More specifically, the arthritogenic peptides may be detected in a lymphocyte sample using anti-arthritogenic peptide antibodies in either liquid or solid phase immunoassay formats (when bound to a carrier). Antibodies for use in these formats may be produced as described *infra*.

Examples of well-known carriers for use in solid-phase assay formats include glass, polystyrene, polypropylene, polyethylene, dextran, nylon, amylases, natural and modified celluloses, polyacrylamides, agaroses and magnetite. The nature of the carrier can be either soluble or insoluble for purposes of the invention.

Examples of types of immunoassays which can utilize anti-arthritogenic antibodies of the invention are competitive and non-competitive immunoassays in either a direct or indirect format. Examples of such immunoassays are the ELISA, the radioimmunoassay (RIA), and the sandwich (immunometric) assay. Binding of arthritogenic peptides using the antibodies of the invention can be done utilizing immunoassays which are run in either the forward, reverse, or simultaneous modes, including immunohistochemical assays on physiological samples. Those of skill in the art will know, or can readily discern other immunoassay formats without undue experimentation.

The protocol described in Example V illustrates how the RA screening method may be performed by immunoassay (there, an enzyme-linked immunoadsorbent assay, or ELISA). In a preferred embodiment of the RA screening method, lymphocytes obtained by conventional means from persons who are suspected of having early stage RA or of being predisposed to the disease are cultured and exposed to immunogenic RA susceptibility peptides, preferably rdnaJ (produced as described *infra*). The cellular immune response of the cultured lymphocytes to the immunogenic dnaJ is then detected. Alternatively, the protocol may be performed as an inhibition study as described in Example IV; i.e., where any reduction in antibody binding through preincubation with dnaJ peptide is detected.

The anti-arthritogenic peptide antibodies used may also be detectably labelled. There are many different labels and methods of labeling known to those of ordinary skill in the art. Examples of the types of labels which can be used in the present invention include enzymes, radioisotopes, fluorescent compounds, colloidal metals, chemiluminescent compounds, and bio-luminescent compounds. Those of ordinary skill in the art will know of other suitable labels for binding to the anti-arthritogenic peptide antibodies of the invention, or will be able to ascertain such, using routine experimentation. Furthermore, the binding of these labels to the anti-arthritogenic peptide antibodies of the invention can be done using standard techniques common to those of ordinary skill in the art. Another labeling technique which may result in greater sensitivity consists of coupling the antibodies to low molecular weight haptens. These haptens can then be specifically detected by means of a second reaction. For example, it is common to use haptens for this purpose such as biotin, which reacts with avidin.

To differentiate between an individual whose HLA-DW4 molecules bear the RA susceptibility sequence peptide but who is nonetheless not predisposed to RA from one who is genetically disposed to RA, the ability of the individual to produce a humoral immune response to immunogenic dnaJ or RA susceptibility sequence peptide may also be tested. To that end, a lymphocyte sample is incubated with an immunogenic arthritogenic peptide. Any anti-arthritogenic peptide antibodies produced in the sample are then detected by an appropriate immunoassay format as described above or in Example V. For convenience of detection, the immunogenic arthritogenic protein or peptide may be detectably labelled as described above.

C. Reagents for Use in the Screening Methods.

Reagents for use in the RA screening method include arthritogenic peptides, preferably those produced synthetically or by recombinant means. Recombinant or synthetic dnaJ proteins and arthritogenic peptides will be particularly preferred reagents for use in the methods of the invention.

1. Methods for Production of Purified, Synthetic and Recombinant

Arthritogenic Peptides

The term "substantially pure" as used herein denotes a protein which is substantially free of other compounds with which it may normally be associated *in vivo*. In the context of the invention, the term refers to homogenous proteins or peptides containing the RA susceptibility sequence, which homogenicity is determined by reference to purity standards known to those of ordinary skill in the art (such as purity sufficient to allow the N-terminal amino acid sequence of the protein to be obtained).

Substantially pure arthritogenic proteins and peptides may be obtained from intact microorganisms (particularly bacteria), through microbial expression, by synthesis, and/or by purification means known to those skilled in the art, such as affinity chromatography. Such techniques may be utilized to obtain immunogenic peptide fragments of dnaJ or other arthritogenic proteins.

The nucleotide and amino acid sequences of several bacterial dnaJ proteins are known. For example, the sequences for *E. coli* dnaJ are reported in Albani, *et al., J.Clin.Invest.,* 89:327-331 (1992) (see also, SEQ ID NO: 1 herein) and the sequences for *Lactococcus lactis* are reported in Van Asseldonk, *et al., J.Bact.,* 175:1637-1644 (1993). With reference to such sequences, dnaJ protein and peptides in particular can be synthesized by such commonly used methods as t-BOC or FMOC protection of alpha-amino groups. Both methods involve stepwise syntheses whereby a single amino acid is added at each step starting from the C terminus of the peptide (see, Coligan, *et al., Current Protocols in Immunology,* Wiley Interscience, 1991, Unit 9). Peptides of the invention can also be synthesized by various well known solid phase peptide synthesis methods, such as those described by Merrifield *(J. Am. Chem. Soc.,* 85:2149, 1962), and Stewart and Young *(Solid Phase Peptides Synthesis,* Freeman, San Francisco, 1969, pp 27-62), using a copoly (styrene-divinylbenzene) containing 0.1-1.0 mMol amines/g polymer. On completion of chemical synthesis, the peptides can be deprotected and cleaved from the polymer by treatment with liquid HF-10% anisole for about 1/4-1 hours at 0°C. After evaporation of the reagents, the peptides are extracted from the polymer with 1% acetic acid solution which is then lyophilized to yield the crude material. This can normally be purified by such techniques as gel filtration on a "SEPHADEX® G-15" or "SEPHAROSE®" affinity column using 5% acetic acid as a solvent. Lyophilization of appropriate fractions of the column will yield the homogeneous peptide or peptide derivatives, which can then be characterized by such standard techniques as amino acid analysis, thin layer chromatography, high performance liquid chromatography, ultraviolet absorption spectroscopy, molar rotation, solubility, and sequenced by the solid phase Edman degradation.

Polynucleotides which encode dnaJ as well as other arthritogenic peptides, and can be used to obtain recombinant, immunogenic forms of these proteins are also provided. As used herein, "polynucleotide" refers to a polymer of deoxyribonucleotides or ribonucleotides, in the form of a separate fragment or as a component of a lager construct. DNA encoding a arthritogenec peptide can be assembled from cDNA fragments or from oligonucleotides which provide a synthetic gene which is capable of being expressed in a recombinant transcriptional unit. Polynucleotide sequences of the invention include DNA, RNA and cDNA sequences. A polynucleotide sequence can be deduced from the genetic code, however, the degeneracy of the code must be taken into account. Polynucleotides of the invention include sequences which are degenerate as a result of the genetic code.

As described in further detail below, polynucleotide sequences encoding arthritogenic proteins can be expressed in either prokaryotes or eukaryotes. Hosts can include microbial yeast, insect and mammalian organisms. Methods of expressing DNA sequences having eukaryotic or viral sequences in prokaryotes are well known in the art. Biologically functional viral and plasmid DNA vectors capable of expression and replication in a host are known in the art. Such vectors (i.e., "recombinant expression vectors") are used to incorporate DNA sequences of the invention. An illustration of the use of an expression vector to express dnaJ is provided in Example I. These sequences may also be contained in "host cells", i.e., transformed cells such as CHO and COS cells for use in gene expression.

DNA encoding RA susceptibility sequence proteins is obtained from sources other than *E. coli.* bacteria by a) obtaining a cDNA library from the particular organism, b) conducting hybridization analysis with labelled DNA encoding *E. coli.* bacterial arthritogenic protein or fragments thereof (usually, greater than 100 bp) in order to detect clones in the cDNA library containing homologous sequences, and c) analyzing the clones by restriction enzyme analysis and nucleic acid sequencing to identify full-length clones. If full length clones are not present in the library, then appropriate fragments may be recovered from the various clones and ligated at restriction sites common to the clones to assemble a full-length clone.

In general, prokaryotes are used for cloning of DNA sequences in constructing dnaJ expressing recombinant expression vectors. For example, *E. coli* K12 strain 294 (ATCC No. 31446) may be particularly useful. Prokaryotes also are used for expression. The aforementioned strain, as well as *E. coli* W3110 (ATTC No. 27325), bacilli such as *Bacillus subtilus,* and other enterobacteriaceae such as *Salmonella typhimurium* or *Serratia marcescans,* and various pseudomonas species may also be used for expression.

In general, plasmid vectors used to produce rdnaJ will contain promoters and control sequences which are derived from species compatible with the host cell. The vector ordinarily carries a replication site as well as marker sequences which are capable of providing phenotypic selection in transformed cells. For example, *E. coli* is typically transformed using pBR322, a plasmid derived from an *E. coli* species (Bolivar, *et al*., *Gene,* 2:95, (1977)). pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA construction.

Promoters suitable for use with prokaryotic hosts illustratively include the β-lactamase and lactose promoter systems (Chang, *et al*., *Nature,* 275:615, 1978; and Goeddel, *et al*., *Nature,* 281:544, 1979), alkaline phosphatase, the tryptophan (trp) promoter system (Goeddel, *Nucleic Acids Res.,* 8:4057, 1980) and hybrid promoters such as the taq promoter (de Boer, *et al., Proc. Natl. Acad. Sci. USA,* 80:21-25, 1983). However, other functional bacterial promoters are suitable. Their nucleotide sequences are generally known in the art, thereby enabling a skilled worker to ligate them to DNA encoding an RA susceptibility sequence protein (Siebenlist, *et al*., *Cell,* 20:269, 1980) using linkers or adapters to supply any required restriction sites.

In addition to prokaryotes, eukaryotic microbes such as yeast cultures may also be used. *Saccharomyces cerevisiae*, or common baker's yeast is the most commonly used eukaryotic microorganism, although a number of other strains are commonly available.

Suitable promoting sequences for use with yeast hosts include the promoters for 3-phosphoglycerate kinase (Hitzeman, *et al., J. Biol. Chem.*, 255:2073, 1980) or other glycolytic enzymes (Hess, et *al. J. Adv. Enzyme Reg.* 7: 149, 1968; and Holland, *Biochemistry*, 17:4900, 1978) such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradatie enzymes associated with nitrogen metabolism, metallothionein, glyceraldehyde-3-phosphate dehydrogenase, and enzymes responsible for maltose and galactose utilization. Yeast enhancers also are advantageously used with yeast promoters.

"Control region" refers to specific sequences at the 5' and 3' ends of eukaryotic genes which may be involved in the control of either transcription or translation. Virtually all eukaryotic genes have an AT-rich region located approximately 25 to 30 bases upstream from the site where transcription is initiated. Another sequence found 70 to 80 bases upstream from the start of transcription of many genes is a CCAATX region where X may be any nucleotide. At the 3' end of most eukaryotic genes is an AATAAA sequence which may be the signal for addition of the poly A tail to the 3' end of the transcribed mRNA.

Preferred promoters controlling transcription from vectors in mammalian host cells may be obtained from various sources, for example, the genomes of viruses such as polyoma, Simian Virus 40 (SV40), adenovirus, retroviruses, hepatitis-B virus and most preferably cytomegalovirus, or from heterologous mammalian promoters, e.g. beta actin promoter. The early and later promoters of the SV40 virus are conveniently obtained as an SV40 restriction fragment which also contains the SV40 viral origin of replication (Fiers, *et al*, *Nature* 273:113, 1978). The immediate early promoter of the human cytomegalovirus is conveniently obtained as a HindIII E restriction fragment (Greenaway, et *al., Gene* 18:355-360, 1982). Promoters from the host cell or related species also are useful herein.

Suitable host cells for transformation with and expression of the vectors of this invention encoding arthritogenic proteins in higher eukaryotes include: monkey kidney CV1 line transformed by SV40 (COS-7, ATCC #1651; human embryonic kidney line (293, Graham, *et al*., *J. Gen Virol*., 36:59, 1977); baby hamster kidney cells (BHK, ATCC CCL 10); chinese hamster ovary-cells-DHFR (DHO, *et al., Proc. Nat'l Sci. Acad.* USA, 77:4216, 1980); mouse sertoli cells (TM4, Mather, J.P., *Biol. Reprod*., 23:243-251, 1980); monkey kidney cells (CV1 ATCC #70); african green monkey kidney cells (VERO-76, ATCC CRL-1587); human cervical carcinoma cells (HELA, ATCC #2); canine kidney cells (MDCK, ATCC #34); buffalo rat liver cells (BRL 3A, ATCC #1442); human lung cells (WI38, ATCC #75); human liver cells (Hep G2, HB 8065); mouse mammary tumor (MMT 060562, ATCC #51); and TRI cells (Mather, et *al., Annals N. Y. Acad. Sci.,* 383:44-68, 1982).

"Transformation" means introducing DNA into an organism so that the DNA is replicable, either as an extrachromosomal element or by chromosomal integration, such as described in Graham, *et al., (Virology,* 52:456-457, 1973). However, other methods for introducing DNA into cells such as by nuclear injection or by protoplast fusion may also be used. If prokaryotic cells or cells which contain substantial cell wall constructions are used, transfection may be achieved by means well known in the art such as calcium treatment using calcium chloride as described by Cohen, F.N., et al., (Proc. Nat'l Acad. Sci. USA, 69:2110, 1972).

"Transfection" refers to the taking up of an expression vector by a host cell whether or not any coding sequences are in fact expressed. Numerous methods of transfection are known to the ordinarily skilled artisan using, for example, $CaPO_4$ or electroporation. Successful transfection is generally recognized when any indication of the operation of the transfected vector occurs within the host cell.

Construction of suitable vectors containing the desired coding and control sequences employ standard ligation techniques. Isolated plasmids or DNA fragments are cleaved, tailored, and relegated using standard techniques in the form desired to form the plasmids required.

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan.

Also of use are polynucleotides which may not encode dnaJ protein but which nonetheless is capable of hybridizing with DNA encoding an arthritogenic protein (particularly at the RA susceptibility sequence) (e.g., "primers" or "probes"). The probes and primers will generally be oligonucleotides. "Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated. Such oligonucleotides may be detectably labelled with a detectable substance such as a fluorescent group, a radioactive atom or a chemiluminescent group by known methods and used in conventional hybridization assays.

The above-described hybridization technique may be adapted to detect whether the RA susceptibility sequence is present in an individual's HLA molecules. Such assays may be employed in identifying the RA susceptibility sequence in an analyte sample (e.g., of HLA molecules), or for in vitro diagnosis such as detection of DNA or mRNA for the RA susceptibility sequence.

Compositions comprising arthritogenic proteins may include such substances as the stabilizers and excipients described below, predetermined amounts of proteins from the cell or organism that served as the source of DNA encoding the growth hormone receptor and binding protein, proteins from other than arthritogenic protein source organisms, and synthetic polypeptides such as poly-L-lysine. Recombinant arthritogenic proteins and peptides which are expressed in allogeneic hosts will of course be expressed completely free of gene source proteins. For example, expression of human polynucleotides in Chinese Hamster Ovary (CHO) cells or other nonhuman higher mammalian cells results in a composition where the protein is not only free of contaminating agents and human proteins.

## 2. Anti-arthritogenic Protein and Anti-RA Susceptibility Sequence Antibodies.

Antibodies for use in the invention may be raised against intact arthritogenic proteins, as well as synthetic or recombinant peptides. Such peptides will include and preferably consist of the RA subsceptibility sequence (hereafter, the "antibodies of the invention"). For example, suitable immunogenic, arthritogenic peptides for use in the method of the invention are described in the Sequence Listing appended hereto as SEQ ID NOs: 4 and 5. The amino acids following the RA susceptibility sequence in SEQ ID NOs: 4 and 5 are likely not required for T-cell activation by the RA susceptibility sequence, but do cause the peptide to be highly charged and, therefore, immunogenic. To that end, preferred immunogenic, arthritogenic peptides for use in producing the antibodies of the invention will be at least about 7-15 amino acids in total length.

Preferably, the antibodies will be raised against recombinant arthritogenic peptides to ensure that the antisera are specific for the RA susceptibility sequence and to enable production of immunogenic peptides on a relatively large scale. Most preferably, the antibodies of the invention will be raised in milk, such as bovine milk, to selectively stimulate production of IgG (thus enhancing the specificity of the antisera for arthritogenic peptides rather than other GI bacterial antigens).

Said antibodies will also be useful to detect the presence of the RA susceptibility sequence on HLA molecules, in populations of E. coli. from the GI tract of an individual, and in certain therapeutic modalities described infra.

To induce an immune response to dnaJ or another arthritogenic protein or peptide in a normal animal, the immunogenecity of the protein or peptide may be enhanced by coupling to a carrier protein by conjugation using techniques which are well-known in the art. Such commonly used materials which are chemically coupled to the molecule to enhance their immunogenecity include keyhole limpet hemocyanin (KLH), thyroglobulin, bovine serum albumin (BSA), and tetanus toxoid. The coupled molecule is then used to immunize the animal (e.g., a mouse, rabbit or cow).

A multiple injection immunization protocol is preferred for use in immunizing animals with immunogenic protein (see, e.g., Langone, *et al.,* eds., "Production of Antisera with Small Doses of Immunogen: Multiple Intradermal Injections", *Methods of Enzymology*, Acad. Press, 1981). For example, an antibody response can be obtained in rabbits by intradermal injection of 1 mg of immunogenic protein emulsified in Complete Freund's Adjuvant followed several weeks later by one or more boosts of the same antigen in incomplete Freund's Adjuvant. A particularly preferred method for production of therapeutic antibodies of the invention in cows is described further *infra*.

Polyclonal antibodies produced by the immunized animals can be further purified, for example, by binding to and elution from a matrix on which the protein or peptide to which the antibodies were raised is bound. Those of skill in the art will know of various techniques common in the immunology arts for purification and/or concentration of polyclonal antibodies, as well as monoclonal antibodies (see, for example, Coligan, *et al.*, *Current Protocols in Immunology,* Unit 9, (Wiley Interscience, 1991)).

For preparation of monoclonal antibodies, immunization of a mouse or rat is preferred. The term "antibody" as used in this invention is meant also to include intact molecules as well as fragments thereof, such as for example, Fab and F(ab')$_2$, which are capable of binding an arthritogenic protein or peptide. Also, in this context, the term "mAb's of the invention" refers to monoclonal antibodies with specificity for an arthritogenic protein or peptide.

The general method used for production of hybridomas secreting monoclonal antibodies ("mAb's") is well known (Kohler and Milstein, Nature, 256:495, 1975). Briefly, as described by Kohler and Milstein, the technique comprised isolation of lymphocytes from regional draining lymph nodes of five separate cancer patients with either melanoma, teratocarcinoma or cancer of the cervix, glioma or lung. The lymphocytes were obtained from surgical specimens, pooled, and then fused with SHFP-1. Hybridomas were screened for production of antibody which bound to cancer cell lines. An equivalent technique can be used to produce and identify mAb's with specificity for an arthritogenic protein.

Confirmation of arthritogenic protein specificity among mAbs of the invention can be accomplished using relatively routine screening techniques (such as the enzyme-linked immunosorbent assay, or "ELISA") to determine the elementary reaction pattern of the mAb of interest.

It is also possible to evaluate an mAb to determine whether is has the same specificity as mAb of the invention without undue experimentation by determining whether the mAb being tested prevents a mAb of the invention from binding to an RA arthritogenic protein. If the mAb being tested competes with the mAb of the invention, as shown by a decrease in binding by the mAb of the invention, then it is likely that the two monoclonal antibodies bind to the same or a closely related epitope.

Still another way to determine whether a mAb has the specificity of a mAb of the invention is to pre-incubate the mAb of the invention with an antigen with which it is normally reactive, and determine if the mAb being tested is inhibited in its ability to bind the antigen. If the mAb being tested is inhibited then, in all likelihood, it binds the same epitope as a mAb of the invention.

III. THERAPEUTIC USES OF THE INVENTION

A. Passive Antibody Therapy.

As noted elsewhere above, it is believed that at least the early stages of RA depend on continual loading of circulating monocytes with IgG immune complexes containing arthritogenic proteins and peptides such as dnaJ. This "loading" is enabled at least in part by the failure of the GI immune system to produce sufficient antibodies, particularly 1gA, to prevent escape of arthritogenic protein, peptides, and/or related antigens into circulation.

One therapeutic use of the invention is therefore directed to antibodies introducable into an individual with early stage RA or who is predisposed to the disease. These antibodies may be produced as described *supra* and will, in that case, preferably be monoclonal antibodies and can be administered enterally, preferably in enteric coated tablet form. Alternately, the antibodies will be produced in milk as described below.

The antibodies used will be specific for dnaJ protein or another arthritogenic protein (hereafter, "therapeutic antibodies"). The antibodies may be raised by immunization of an animal with these antigens in native, synthetic or recombinant form. The antigens used may be derived from any organism which expresses the RA susceptibility sequence, but will preferably be raised against microorganisms which reside in the gut and express the peptide as part of a heat shock protein. Most preferably, the antigens will be derived from *E. coli.* (due to the prevalence of this species in the human GI tract), but may also be raised against a cocktail of antigens from different microbial species to ensure that the antibodies will be specific for arthritogenic proteins and peptides from a broad spectrum of organisms.

To develop antibodies in milk (which may then be administered therapeutically as "immune milk"), lactating mammals, preferably cows, are immunized with immunogenic RA susceptibility sequence peptide as generally described *supra*. Cow's milk is the preferred medium for production of anti-RA susceptibility sequence peptide antibodies due to its absorbability into the human GI tract. The milk may be collected, pasteurized and, preferably, lyophilized for storage by conventional techniques and used as a dry milk powder. The immune milk powder may be administered in powder or reconstituted form by enteral routes generally as described in U.S. Patent No. 4,732,737 (from column 8, line 5 through column 14, line 11).

B. Antibiotic Elimination and Repopulation of Gut Bacteria

Although proteins do not normally efficiently cross the intestinal barrier in humans, it appears based on the observations discussed elsewhere above that arthritogenic antigens (e.g., dnaJ) do come into contact with the systemic immune system of persons with RA. This embodiment of the invention is therefore directed toward preventing or minimizing contact between the systemic immune system and arthritogenic antigens by eliminating the source of the latter. More specifically, according to this method of the invention, GI tract bacteria which may express the RA susceptibility sequence as part of a heat shock protein are replaced with otherwise identical bacteria which are incapable of expressing the RA susceptibility sequence (i.e., "RA benign bacteria", which may either contain an "RA benign susceptibility sequence", or may be free of the RA susceptibility sequence).

Conveniently, the RA benign bacteria will be generated by site-specific mutagenesis of the RA susceptibility sequence. Comparisons of the amino acid sequence in dnaJ proteins from various microbial species strongly suggest that the RA susceptibility sequence is not essential to the biological activity of the protein (see, e.g., Silver, *et al., Cell,* 74: 5-6 (1993)). Thus, modification of the RA susceptibility sequence in a microorganism to render the expression product benign should not impair the ability of the organism to survive in the human GI tract.

However, a modification of the RA susceptibility sequence from QKRAA to DERAA substantially reduces the ability of the modified arthritogenic protein to stimulate lymphocytes from individuals with early stage RA (see, Example III and FIGURE 2). Thus, the modification of the RA susceptibility sequence from QK/RRAA to DERRAA or an equivalent variant will be a preferred modification which will generate the RA benign bacteria of the invention.

Other minor modifications of the primary amino acid sequence of dnaJ, or of other arthritogenic proteins, may result in variants which are RA benign but have otherwise substantially equivalent activity to the wild-type protein. All of the variants produced by these modifications are included herein as long as the resulting, modified microorganism is RA benign and the biological activity present in the original protein otherwise still exists.

Amino acid sequence variants fall into one or more of three classes; substitutional, insertional or deletional variants. The variants ordinarily are prepared by site specific mutagenesis of nucleotides in the DNA encoding the wild-type protein, thereby producing DNA encoding the variant, and thereafter expressing the DNA in recombinant cell culture. However, peptides having up to about 100-150 residues may be conveniently prepared by *in vitro* synthesis.

While the site for introducing an amino acid sequence variation is predetermined, the mutation per se need not be predetermined. For example, in order to optimize the performance of a mutation at a given site, random mutagenesis may be directed at the target codon or region and the expressed RA benign susceptibility sequence peptides variants screened for the optimal combination of desired activity. Techniques for making substitution mutations at predetermined sites in DNA having a known sequence are well known, for example M13 primer mutagenesis. Amino acid substitutions are typically of single residues; insertions usually will be on the order of about from 1 to 10 amino acid residues; and deletions will range about from 1 to 30 residues. Deletions or insertions preferably are made in adjacent pairs, i.e., a deletion of 2 residues or insertion of 2 residues.

In addition to rendering the RA susceptibility sequence RA benign, modifications may be made to increase or decrease the stability of the protein, to impair its expression (by, for example, disabling a promoter which would normally dictate expression of the RA susceptibility sequence), or to accomplish other desirable modifications in the protein, which modifications will be apparent to those of ordinary skill in the art. For example, deletion or substitution of potential proteolysis sites can also be accomplished by deleting such residues or substituting a glutaminyl or histidyl residue. Such modifications to intact bacteria may be accomplished by conventional recombinant DNA technology, including the techniques described in Section II, C, supra.

Most preferably, the RA benign bacteria will be resistant to antibiotics. To this end, a polynucleotide conferring such antibiotic resistance will be included in the genome of the RA benign bacteria, such as the genes which confer ampicillin and tetracycline resistance which are well-known in the art (see, e.g., Bolivar, et *al*., *Gene, 2*:95 (1977)). Most preferably, the RA benign bacteria of the invention will also be rendered substantially free of lipopolysaccharide ("LPS") by means well-known in the art (see, e.g., *J.Immun.Methods*, 132: 191-195, 1990).

The RA benign bacteria will be used to repopulate a human GI tract in which the endogenous bacterial population (predominantly *E. coli*.) has been depleted. This depletion may be accomplished by intensive antibiotic therapy accomplished with means and reagents well-known to those skilled in the clinical arts. A particularly efficacious therapeutic regime for use in this regard would involve administering a nonabsorbable antibiotic preferably a broad spectrum antibiotic, (such as neomycin or kanamycin) in therapeutically effective dosages by enteral routes to an individual with early stage RA or who is predisposed to the disease for a period of one to eight weeks or longer. In this context, a "therapeutically effective dosage" of antibiotic will be one which is sufficient to substantially eliminate the endogenous bacterial population from the GI tract of the patient. Such dosages will vary depending on the antibiotic administered; suitable dosages will be known to physicians of ordinary skill in the art. The extent to which RA benign bacteria can be administered will depend on the age, weight and condition of the patient as well as the extent to which the endogenous bacterial population they will replace is depleted. However, a therapeutically effective dosage of RA benign bacteria will generally be $10^6$ to $10^8$ bacteria/dose.

To minimize adverse reactions in the patient from depletion of the endogenous bacterial population of the patient's GI tract during the course of the antibiotic therapy, the antibiotics may be administered under substantially sterile conditions in a hospital setting or with appropriate limitations on the patient's activities and diet. Alternatively or concurrently, a more practical therapeutic approach would introduce antibiotic-resistant RA benign bacteria to the patient's GI tract during the course of the antibiotic therapy to replace the antibiotic depleted population of wild-type bacteria.

C. Active Immunization with an RA Susceptibility Sequence Protein

The above-described uses according to the invention may be supplemented with an active immunization protocol whereby the patient is immunized with an immunogenic RA susceptibility sequence protein or peptide. Further, to ensure purity and reproducability of the immunizing peptide, it will preferably be produced and used in recombinant form.

Recombinant arthritogenic proteins may be produced as described elsewhere above and as illustrated in Example I, *infra*. Immunogenic forms of these proteins and conjugates thereof may be produced as described above and used as a vaccine ("RA vaccine") generally as described with respect to synthetic RA susceptibility sequence vaccines in PCT Published Patent Application No. WO 9014835, "Method to Treat Rheumatoid Arthritis", Carson, *et al.,* inventors (filed 5/31/90).

More specifically, the immunogenic peptides can preferably be administered as an RA vaccine in a manner sufficient to selectively stimulate IgA production in the patient's GI tract. Therefore, the immunogenic peptides can preferably be administered concomitantly with an immunostimulant which is known in the art to induce IgM switching to IgA; e.g., TGF-β, which may also inhibit systemic helper T cell activity. Most preferably, the immunogenic peptide/ IgA immunostimulant of the invention can be administered enternally to localize the effect of the vaccine in the GI tract.

Depending on the patient's presenting condition, it may also be desirable to administer immunostimulants and/or immunosuppressants whose action will be directed to the patient's systemic immune system. Suitable substances having this activity are well-known in the art and include interleukin-6 (for stimulation of suppressor/cytotoxic T cells) as well as cyclosporin A and anti-CD4 antibodies (for immune suppression). These substances may be administered according to the judgment of a skilled clinician to preferentially expand the suppressor/cytotoxic T lymphocyte population of a patient whose systemic immune system is suspected of being, or known to have been, exposed to an arthritogenic antigen.

The RA vaccines of the invention may be prepared for administration by mixing the active components of the vaccine with physiologically acceptable carriers. Such carriers will be nontoxic to recipients at the dosages and concentrations employed. Ordinarily, the preparation of such compositions entails combining the particular protein with buffers, antioxidants such as ascorbic acid, low molecular weight (less than about 10 residues) polypeptides, proteins, amino acids, carbohydrates including glucose or dextrins, chelating agents such as EDTA, glutathione and other stabilizers and excipients. Such compositions may be in suspension, emulsion or lyophilized form and will be pharmaceutically acceptable; i.e., suitably prepared and approved for use in the desired application.

With respect to compositions containing TGF-β, molecules of the TGF-β family are ordinarily produced in the form of inert precursors that are activated by exposure to acid or proteases. Those of ordinary skill in the art will, therefore, be able to select carriers and/or excipients for use in the RA vaccine compositions of the invention which will neither interfere with the *in vivo* activation of the TGF-β component or activate the component prematurely (i.e., prior to administration of the composition).

The protocol for administration of the RA vaccines of the invention will vary with their composition as well as the age, weight and condition of the patient. However, a preferred protocol for administration of the RA vaccines will involve daily oral (enteral) administration of immunologically effective dosages of the vaccine, with or without other forms of therapy, such as the methods described above and conventional anti-inflammatory treatments (e.g., use of steroidal or nonsteroidal anti-inflammatory medicaments and/or analgesics).

Depending on the frequency of dosage, an immunologically effective dosage will range from 100 μg-100 mg (preferably 1-100 mg) immunogenic protein or peptide. Daily dosages of immunogenic peptide will most preferably be given in amounts of about 1mg/day. Conventional dosages of any immunostimulant/immuno-suppressant component in the RA vaccine will be used (e.g., 1-100 μg/kg interleukin-6). Such dosages will be known to, or may be readily ascertained by, those of ordinary skill in the art. Treatment in early stage RA patients may be continued through to or beyond observation of the surrogate end-point for RA described further below.

Vaccination of patients who are predisposed to RA, but have not developed the disease, may be accomplished by short-term administration of one or more dosages of the RA vaccines of the invention sufficient to produce detectable increases in anti-immunogenic peptide IgA in fluids of the patient's GI tract. Generally, however, the preferred use of the RA vaccines of the invention will be in patients with early stage RA.

D. Indicia of Therapeutic Efficacy for the uses according to the Invention.

Generally, the efficacy of the therapy according to the invention over time may be judged by an absence of clinical signs of RA in patients who are predisposed to the disease (but have not developed it). In patients who have been diagnosed as having RA, the efficacy of the therapy will be measured by a lessening in the severity of a patient's symptoms or by the occurrence of a surrogate end-point for the disease.

Conventional parameters for clinical symptoms of RA and surrogate end-points for the disease are well-known to those of ordinary skill in the art, including "Ritchie Index" measurements for joint tenderness (Ritchie, *et al.*, *Q.J. Med,* 37: 393-406 (1968)), the number of swollen joints, daily duration of morning joint stiffness, grip strength, intensity of pain on a visual analogue scale (Huskisson, *Lancet,* 2:1127-1131 (1974)), and relative levels of use of anti-inflammatory agents and/or analgesics by the patient.

Based on the results of double-blind clinical trials of an *E. coli.* extract, Brackertz, *et al.*, *J. Rheum.* (16:19-23 (1989)) one of ordinary skill in the art would expect clinical tolerance of the RA vaccine and antibody compositions to be good. The severity of the most possible side effects (such as gastrointestinal irritation) would be expected to be low. Toxicity of these compositions may be monitored by conventional means, such as periodic laboratory evaluations through assays of such variables as hematocrit, hemoglobin, thrombocyte and rheumatoid factor levels as well as blood chemistry.

The invention having been fully described, it is illustrated by examples below. The examples will, however, be understood as exemplifying rather than limiting the scope of the invention.

## EXAMPLE I

## CLONING, EXPRESSION AND PURIFICATION OF *E. Coli*

A 1.1-kb DNA fragment containing the *E. coli* dnaJ gene (see, SEQ ID NO: 1) was amplified by polymerase chain reaction from a recombinant phage encompassing the dnaK and dnaJ genes of *E. coli*. The polymerase chain reaction product was subcloned in the Xba 1 site of the vector PCG808fX (New England Biolabs, Beverly, MA) and expressed as a maltose binding fusion protein that was purified by filtration on any amylose column and elution in an amylose buffer. A rabbit antiserum to the fusion protein was used to purify rdnaJ expressed by cloning in the Xba 1 site of a pUC 19 vector. To this end, rabbit IgG were purified on immobilized protein A (Pierce Chemical Co., Rockford, IL) and coupled to an agarose support matrix (AFFI-GEL Hz, a trademarked product of Bio-Rad Laboratories, Richmond, CA). ʳdnaJ was then obtained by running the raw cellular extracts through the affinity column and eluting the bound protein in glycine buffer, pH 2.5. Polyacrylamide gel electrophoresis of the purified product showed one unique band with the expected molecular mass of 41 kD.

## EXAMPLE II

## RESPONSE OF LYMPHOCYTES FROM PATIENTS WITH EARLY STAGE RA TO rdnaJ

Lymphocytes from patients with early untreated rheumatoid arthritis (RA) were obtained from blood (peripheral blood lymphocytes, or "PBL's") for from synovial fluids using standard aseptic techniques. The cells were incubated for 7 days with purified recombinant *E. coli* dnaJ (10 μg/ml, obtained as described in Example I) in RPMI 1640 medium supplemented with 10% prescreened human AB type serum. Lymphocytes from 10 control subjects (i.e., persons without RA) were treated similarly. Proliferation during the last 16 hours of culture was expressed as a

$$\text{stimulation index} = \frac{\text{cpm in cultures with dnaJ}}{\text{cpm in cultures without dnaJ.}}$$

Based on cellular proliferation responses of PBL's from 14 early stage RA patients, and of synovial fluid lymphocytes from 8 early stage RA, the proliferation responses of the PBL's was approximately 3.6 times greater than the response of control subject lymphocytes while the response of synovial fluid lymphocytes was approximately 2.3 times greater than the response of control subject lymphocytes (see, FIGURE 1).

**EXAMPLE III**

**RESPONSE OF LYMPHOCYTES FROM PATIENTS WITH EARLY STAGE RA TO RA SUSCEPTIBILITY SEQUENCE PEPTIDES**

Lymphocytes from patients with early stage RA were contacted with one of two RA susceptibility sequence peptides according to the protocol described in Example II. The peptides used were either a 15 amino acid synthetic peptide having the wild-type sequence QKRAAYDQYGHAAFE or a 15 amino acid synthetic mutant peptide having the sequence DERAAYDQYGHAAFE.

As shown in FIGURE 2, gauging the results according to the same standard of measurement used for Example II, the responses of PBL's to the wild-type peptide were more than 5 times greater than the responses of PBL's to the mutant peptide and approximately 9 times greater than the response of lymphocytes from control subjects to the wild-type peptide.

**EXAMPLE IV**

**INHIBITION OF ANTIBODY BINDING TO dnaJ**

Microtiter plates were coated with recombinant dnaJ (10μg/ml). Either sera obtained from patients with early stage RA or sera from control subjects were diluted 1:100 in borate buffered saline. Half of each specimen was incubated overnight at 4°C with the wild-type dnaJ peptide described in Example III. Then the paired serum specimens were added to the dnaJ coated plates.

After 4 hours incubation, antibody binding was detected with alkaline phosphatase labelled goat anti-human IgG; levels of detected antibody binding were determined according to the following formula:

$$\% \text{ inhibition} = 100 \; \frac{(1 - OD_{460} \text{ with wild-type peptide})}{(OD_{460} \text{without wild-type peptide})}$$

As shown in FIGURE 3, antibody binding to rdnaJ was inhibited to, by the wild-type RA susceptibility sequence peptide, approximately a 3 times greater extent in the RA sera than the inhibition detected in the control sera.

**EXAMPLE V**

**DETERMINATION OF GENETIC PREDISPOSITION TO RA BY IMMUNOASSAY**

Antibody responses to rdnaJ were measured by ELISA. 100μl aliquots of $^r$dnaJ (10 μg/ml) or membrane proteins from HLA DR or DR41 homozygous cells (100 μg/ml) were used to coat ELISA plates at 4°C overnight. Then different dilutions of rabbit sera were added for a 2 hour incubation at room temperature. After washing with BBS/0.2% Tween-20, bound antibody was detected by using alkaline phosphatase-conjugated goat anti-rabbit IgG (Boehringer-Mannheim Biochemicals) diluted 1:1000. The results are expressed in FIGURE 4 as the mean OD at 405 nm of duplicate wells.

**SUMMARY OF SEQUENCES**

SEQ ID NO:1 is the open reading frame of the polynucleotide encoding *E. coli* K12 dnaJ.
SEQ ID NO:2 is the amino acid sequence of a RA susceptibility sequence variant.
SEQ ID NO:3 is the amino acid sequence of a RA susceptibility sequence variant.
SEQ ID NO:4 is the amino acid sequence for an immunogenic dnaJ peptide.
SEQ ID NO:5 is the amino acid sequence for an immunogenic dnaJ peptide.

**Claims**

1.  Use of a nonabsorbable antibiotic or rheumatoid arthritis (RA) benign bacteria for the manufacture of a medicament for reducing exposure to arthritogenic peptides of a person who has RA or is predisposed to develop RA, wherein said medicament consists of

    a: a nonabsorbable antibiotic to be administered enterally to the person so as to reduce the population of bacteria that express a RA susceptibility sequence; and
    b: RA benign bacteria to be administered to the person, thereby reducing exposure of the person to arthritogenic peptides.

2.  The use according to claim 1, wherein the RA benign bacteria are to be administered in an amount of about $10^6$ to $10^8$ bacteria per dose.

3.  The use according to claim 1, wherein the RA benign bacteria are selected from genera consisting of *Escherichia, Lactococcus, Klebsiella, and Proteus.*

4.  The use according to claim 1, wherein the genetic predisposition to RA is determined by detecting the presence of the RA susceptibility sequence on the person's HLA molecules.

5.  The use according to claim 1, wherein the person has early stage RA.

6.  Use of an anti-arthritogenic antibody that binds specifically to one or more arthritogenic peptides, for the manufacture of a medicament for reducing exposure of a person who has RA or is predisposed to develop RA wherein said antibody is to be administered enterally to the person.

7.  The use according to claim 6, wherein the anti-arthritogenic antibody is to be administered in milk.

8.  The use according to claim 7, wherein the anti-arthritogenic antibody is produced in milk.

9.  The use according to claim 6, wherein the anti-arthritogenic antibody is a monoclonal antibody that specifically binds the RA susceptibility sequence of dnaJ.

10. The use according to claim 9, wherein the dnaJ is produced by bacteria selected from at least one of the genera *Escherichia, Lactococcus, Klebsiella, Proteus, and Salmonella.*

11. The use according to claim 6, wherein the genetic predisposition to RA is determined by detecting the presence of the RA susceptibility sequence on the person's HLA molecules.

12. The use of claim 1 in combination with the use of claim 6 for reducing exposure to arthritogenic peptides of a person who has RA or is genetically disposed to develop RA.

13. The use according to claim 1, 6 or 12, wherein the medicament further comprises immunologically effective recombinant, anti-arthritogenic peptides.

14. The use according to claim 13, wherein the anti-arthritogenic peptides are to be administered concomitantly with immunostimulatory or immunosuppressant agents.

**Patentansprüche**

1. Verwendung von einem nicht absorbierbaren Antibiotikum oder von im Hinblick auf rheumatoide Arthritis (RA) unschädlichen Bakterien zur Herstellung eines Arzneimittels zum Vermindern der Exposition einer Person, die RA hat oder prädisponiert ist, RA zu entwickeln, gegenüber arthritogenen Peptiden, worin das Arzneimittel besteht aus:

   a: einem nicht absorbierbaren Antibiotikum, das der Person enteral zu verabreichen ist, um die Bakterienpopulation, die eine RA-Anfälligkeitssequenz exprimiert, zu vermindern; und

   b: im Hinblick auf RA unschädliche Bakterien, die der Person zu verabreichen sind, wodurch die Exposition der Person gegenüber arthritogenen Peptiden vermindert wird.

2. Die Verwendung gemäß Anspruch 1, worin die im Hinblick auf RA unschädlichen Bakterien in einer Menge von etwa $10^6$ bis $10^8$ Bakterien pro Dosis zu verabreichen sind.

3. Die Verwendung gemäß Anspruch 1, worin die im Hinblick auf RA unschädlichen Bakterien ausgewählt werden aus Gattungen bestehend aus *Escherichia, Lactococcus, Klebsiella* und *Proteus*.

4. Die Verwendung gemäß Anspruch 1, worin die genetische Prädisposition für RA bestimmt wird durch Detektion des Vorliegens der RA-Anfälligkeitssequenz auf den HLA-Molekülen der Person.

5. Die Verwendung gemäß Anspruch 1, worin die Person RA im frühen Stadium hat.

6. Verwendung eines anti-arthritogenen Antikörpers, der spezifisch an ein oder mehrere arthritogene Peptide bindet, zur Herstellung eines Arzneimittels zum Vermindern der Exposition einer Person, die RA hat oder prädisponiert, RA zu entwickeln, gegenüber arthritogenen Peptiden, worin der Antikörper der Person enteral zu verabreichen ist.

7. Die Verwendung gemäß Anspruch 6, worin der anti-arthritogene Antikörper in Milch zu verabreichen ist.

8. Die Verwendung gemäß Anspruch 7, worin der anti-arthritogene Antikörper in Milch produziert wird.

9. Die Verwendung gemäß Anspruch 6, worin der anti-arthritogene Antikörper ein monoklonaler Antikörper ist, der spezifisch an die RA-Anfälligkeitssequenz von dnaJ bindet.

10. Die Verwendung gemäß Anspruch 9, worin dnaJ durch Bakterien produziert wird, ausgewählt aus wenigstens einer der Gattungen *Escherichia, Lactococcus, Klebsiella*, *Proteus* und *Salmonella*.

11. Die Verwendung gemäß Anspruch 6, worin die genetische Prädisposition für RA bestimmt wird durch Detektion des Vorliegens der RA-Anfälligkeitssequenz auf den HLA-Molekülen der Person.

12. Die Verwendung gemäß Anspruch 1 in Kombination mit der Verwendung gemäß Anspruch 6 zum Vermindern der Exposition einer Person, die RA hat oder genetisch prädisponiert ist, RA zu entwickeln, gegenüber arthritogenen Peptiden.

13. Die Verwendung gemäß Anspruch 1, 6 oder 12, worin das Arzneimittel zusätzlich immunologisch wirksame rekombinante anti-arthritogene Peptide umfasst.

14. Die Verwendung gemäß Anspruch 13, worin die anti-arthritogenen Peptide gleichzeitig mit immunstimulierenden oder immunsupprimierenden Mitteln zu verabreichen sind.

**Revendications**

1. Utilisation d'un antibiotique non-absorbable ou de bactéries bénignes de l'arthrite rhumatoïde (AR) pour la préparation d'un médicament pour réduire l'exposition à des peptides arthritogènes d'une personne qui a de l'arthrite rhumatoïde ou qui est prédisposée à développer de l'arthrite rhumatoïde, ledit médicament étant constitué de :

   a) un antibiotique non-absorbable, à administrer par voie entérale à la personne afin de réduire la population des bactéries qui expriment une séquence de sensibilité à l'arthrite rhumatoïde, et
   b) des bactéries bénignes de l'arthrite rhumatoïde, à administrer à la personne, ce qui réduit l'exposition de la personne à des peptides arthritogènes.

2. Utilisation selon la revendication 1, dans laquelle les bactéries bénignes de AR sont à administrer en une quantité d'environ $10^6$ à environ $10^8$ bactéries par dose.

3. Utilisation selon la revendication 1, dans laquelle les bactéries bénignes de AR sont choisies parmi les genres *Escherichia, Lactococcus, Klebsiella* et *Proteus*.

4. Utilisation selon la revendication 1, dans laquelle la prédisposition génétique à AR est déterminée par détection de la présence de la séquence de sensibilité à AR sur les molécules de HLA de la personne.

5. Utilisation selon la revendication 1, dans laquelle la personne a une AR à un stade de début.

6. Utilisation d'un anticorps anti-arthritogène qui se lie spécifiquement à un ou plusieurs peptides arthritogènes, pour la préparation d'un médicament pour réduire l'exposition d'une personne qui a de l'arthrite rhumatoïde ou qui est prédisposée à développer de l'arthrite rhumatoïde, dans laquelle ledit anticorps est à administrer par voie entérale à la personne.

7. Utilisation selon la revendication 6, dans laquelle l'anticorps anti-arthritogène est à administrer dans du lait.

8. Utilisation selon la revendication 7, dans laquelle l'anticorps anti-arthritogène est produit dans du lait.

9. Utilisation selon la revendication 6, dans laquelle l'anticorps anti-arthritogène est un anticorps monoclonal qui se lie spécifiquement à la séquence de sensibilité à AR de dnaJ.

10. Utilisation selon la revendication 9, dans laquelle le dnaJ est produit par des bactéries choisies parmi au moins l'un des genres *Escherichia, Lactococcus, Klebsiella, Proteus* et *Salmonella.*

11. Utilisation selon la revendication 6, dans laquelle la prédisposition génétique à AR est déterminée par détection de la présence de la séquence de sensibilité à AR sur les molécule de HLA de la personne.

12. Utilisation selon la revendication 1, en combinaison avec l'utilisation selon la revendication 6, pour réduire l'exposition à des peptides arthritogènes d'une personne qui a de l'arthrite rhumatoïde ou qui est prédisposée génétiquement à développer de l'arthrite rhumatoïde.

13. Utilisation selon la revendication 1, 6 ou 12, dans laquelle le médicament comprend en outre des peptides anti-arthritogènes, recombinants, immunologiquement efficaces.

14. Utilisation selon la revendication 13, dans laquelle les peptides anti-arthritogènes sont à administrer en même temps que des agents immunostimulants ou immunosuppresseurs.

# FIG. 1

CELLULAR RESPONSES TO dnaJ

STIMULATION INDEX

RA PBLs
n = 14
p<0.02

SYNOV. FLUID CELLS
n = 8
p<0.02

CONTROLS
n = 10

# FIG. 4

anti r dnaj
anti r dnaj pre

1/50    1/100    1/200    1/100

SERUM DILUTIONS

INHIBITION rdna J

## FIG. 2

PROLIFERATION TO dnaJ PEPTIDES

STIMULATION INDEX

20

10

0

RA PBLs
n = 14
NATIVE PEPTIDE
p<0.005

SYNOV. CELLS
n = 8
NATIVE PEPTIDE
p<0.01

RA PBLs
n= 4
MUTATED
PEPTIDE

CONTROLS PBLs
n = 10
NATIVE PEPTIDE

## FIG. 3

DnaJ INHIBITION STUDIES

% INHIBITION

50

40

30

20

10

0

RA PATIENTS
n = 51

CONTROLS
n = 37

p<0.0001

SEQUENCE LISTING

    (1) GENERAL INFORMATION:

        (i) APPLICANT: THE REGENTS OF THE UNIVERSITY OF CALIFORNIA

(ii) TITLE OF INVENTION: METHOD AND REAGENTS FOR THE TREATMENT OF RHEUMATOID ARTHRITIS

(iii) NUMBER OF SEQUENCES: 5

(iv) CORRESPONDENCE ADDRESS:

    (A) ADDRESSEE: ROBBINS, BERLINER & CARSON
    (B) STREET: 201 N. FIGUEROA STREET, 5TH FLOOR
    (C) CITY: LOS ANGELES
    (D) STATE: CALIFORNIA
    (E) COUNTRY: US
    (F) ZIP: 90012

(v) COMPUTER READABLE FORM:

    (A) MEDIUM TYPE: Floppy disk
    (B) COMPUTER: IBM PC compatible
    (C) OPERATING SYSTEM: PC-DOS/MS-DOS
    (D) SOFTWARE: PatentIn Release #1.0, Version #1.25

(vi) CURRENT APPLICATION DATA:

    (A) APPLICATION NUMBER:
    (B) FILING DATE:
    (C) CLASSIFICATION:

(viii) ATTORNEY/AGENT INFORMATION:

    (A) NAME: BERLINER, ROBERT
    (B) REGISTRATION NUMBER: 20,121
    (C) REFERENCE/DOCKET NUMBER: 5555-314

(ix) TELECOMMUNICATION INFORMATION:

    (A) TELEPHONE: 213-977-1001
    (B) TELEFAX: 213-977-1003

(2) INFORMATION FOR SEQ ID NO:1:

(i) SEQUENCE CHARACTERISTICS:

    (A) LENGTH: 1055 base pairs
    (B) TYPE: nucleic acid
    (C) STRANDEDNESS: single
    (D) TOPOLOGY: linear

(ii) MOLECULE TYPE: DNA (clonal)

(vii) IMMEDIATE SOURCE:

    (B) CLONE: E. coli K12 dnaJ polynucleotide (cDNA)

(ix) FEATURE:

    (A) NAME/KEY: CDS
    (B) LOCATION: 1..1054

(xi) SEQUENCE DESCRIPTION: SEQ ID NO:1:

```
ATGGCTAAGC AAGATTATTA CGAGATTTTA GGCGTTTCCA AAACAGCGGA AGAGCGTGAA        60
ATCAGAAAGG CCTACAAACG CCTGGCCATG AAATACCACC CGGACCGTAA CCAGGGTGAC       120
AAAGAGGCCT ATGGTCATGC TGCGTTTGAG CAAGGTGGCA TGGGCGGCGG CGGTTTTGGC       180
GGCGGCGCAG ACTTCAGCGA TATTTTTGGT GACGTTTTCG GCGATATTTT TGGCGGCGGA       240
CGTGGTCGTC AACGTGCGGC GCGCGGTGCT GATTTACGCT ATAACATGGA GCTCACCCTC       300
GAAGAAGCTG TACGTGGCGT GACCAAAGAG ATCCGCATTC CGACTCTGGA AGAGTGTGAC       360

GTTTGCCACG GTAGCGGTGC AAAACCAGGT ACACAGCCGC AGACTTGTCC GACCTGTCAT       420
GGTTCTGGTC AGGTGCAGAG GCGCCAGGGA TTCTTCGCTG RACAGCAGAC CTGTCAACAC       480
TGTCAGGGCC GCGGTACGCT GATCAAAGAT CCGTGTAACA AATGTCATGG TCATGGTCGT       540
CTTGAGCGCA GCAAAACGCT TCCGTTAAAA TCCCGGCAGG GGTGGACACT GGACACCGCA       600
TCCGTCTTGC GGGCGAAGGT GAAGCGGGCG AGCATGGCGC ACCGGCAGGC GATCTGTACG       660
TTCAGGTTCA GGTTAAACAG CACCCGATTT TCGAGCGTGA AGGCAACAAC CTGTATTGCG       720
AAGTCCCGAT CAACTTCGCT ATGGCGGCGC TGGGTGGCGA AATCGAAGTA CCGACCCTTG       780
ATGGTCGCGT CAAACTGAAA GTGCCTGGCG AAACCCAGAC CGGTAAGCTA TTCCGTATGC       840
GCGGTAAAGG CGTCAAGTCT GTCCGCGGTG GCGCACAGGG TGATTTGCTG TGCCGCGTTG       900
TCGTCGAAAC ACCGGTAGGC CTGAACGAAA GGCAGAAACA GCTGCTGCAA GAGGTGCAAG       960
AAAGATTCGG TGGCCCAACC GGCGAGCACA ACAGCCCGCG CTCAAAGAGC TTCTTTGATG      1020
GTGTGAAGAA GTTTTTTGAC GACCTGACCC GCTAA                                  1055
```

(2) INFORMATION FOR SEQ ID NO:2:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(vii) IMMEDIATE SOURCE:

(B) CLONE: RA Susceptibility Sequence Variant

(ix) FEATURE:

(A) NAME/KEY: Peptide
(B) LOCATION: 1..5

(xi) SEQUENCE DESCRIPTION: SEQ ID NO:2:

```
Gln Lys Arg Arg Ala
 1               5
```

(2) INFORMATION FOR SEQ ID NO:3,

   (i) SEQUENCE CHARACTERISTICS:

      (A) LENGTH: 5 amino acids
      (B) TYPE: amino acid
      (C) STRANDEDNESS: single
      (D) TOPOLOGY: linear

   (ii) MOLECULE TYPE: peptide

   (vii) IMMEDIATE SOURCE:

      (B) CLONE: RA Susceptibility Sequence Variant

   (ix) FEATURE:

      (A) NAME/KEY: Peptide
      (B) LOCATION: 1..5

   (xi) SEQUENCE DESCRIPTION: SEQ ID NO:3:

```
                              Gln Arg Arg Ala Ala
                              1               5
```

(2) INFORMATION FOR SEQ ID NO:4:

   (i) SEQUENCE CHARACTERISTICS:

      (A) LENGTH: 15 amino acids
      (B) TYPE: amino acid
      (C) STRANDEDNESS: single
      (D) TOPOLOGY: linear

   (ii) MOLECULE TYPE: peptide

   (vii) IMMEDIATE SOURCE:

      (B) CLONE: Immunogenic dnaJ Peptide

   (ix) FEATURE:

      (A) NAME/KEY: Peptide
      (B) LOCATION: 1..15

   (xi) SEQUENCE DESCRIPTION: SEQ ID NO:4:

```
        Gln Lys Arg Ala Ala Tyr Asp Gln Tyr Gly His Ala Ala Phe Glu
        1               5               10              15
```

(2) INFORMATION FOR SEQ ID NO:5:

   (i) SEQUENCE CHARACTERISTICS:

      (A) LENGTH: 15 amino acids
      (B) TYPE: amino acid

(C) STRANDEDNESS: single
(D) TOPOLOGY: linear

(ii) MOLECULE TYPE: peptide

(vii) IMMEDIATE SOURCE:

(B) CLONE: Immunogenic dnaJ Peptide

(ix) FEATURE:

(A) NAME/KEY: Peptide
(B) LOCATION: 1..15

(xi) SEQUENCE DESCRIPTION: SEQ ID NO:5:

```
Asp Glu Arg Ala Ala Tyr Asp Gln Tyr Gly His Ala Ala Phe Glu
1               5                   10                  15
```